(19) Europäisches Patentamt
European Patent Office
Office européen des brevets

(11) Veröffentlichungsnummer : **0 456 125 B1**

(12) **EUROPÄISCHE PATENTSCHRIFT**

(45) Veröffentlichungstag der Patentschrift : **27.07.94 Patentblatt 94/30**

(51) Int. Cl.$^5$ : **C07D 403/10,** C07D 403/14, A01N 43/50, A01N 43/56, A01N 43/653

(21) Anmeldenummer : **91107189.2**

(22) Anmeldetag : **03.05.91**

(54) **N-Aryltetrahydrophthalimide und ihre Verwendung als Herbizide.**

(30) Priorität : **11.05.90 DE 4015144**

(43) Veröffentlichungstag der Anmeldung : **13.11.91 Patentblatt 91/46**

(45) Bekanntmachung des Hinweises auf die Patenterteilung : **27.07.94 Patentblatt 94/30**

(84) Benannte Vertragsstaaten : **CH DE FR GB IT LI NL**

(56) Entgegenhaltungen :
**EP-A- 0 233 151**
**EP-A- 0 337 151**

(73) Patentinhaber : **BASF Aktiengesellschaft**
**Carl-Bosch-Strasse 38**
**D-67063 Ludwigshafen (DE)**

(72) Erfinder : **Seele, Rainer, Dr.**
**Leiblstrasse 3**
**W-6701 Fussgoenheim (DE)**
Erfinder : **Rueb, Lothar, Dr.**
**Am Schoeneck 11**
**W-6720 Speyer (DE)**
Erfinder : **Kober, Reiner, Dr.**
**Im Schlittweg 20**
**W-6701 Fussgoenheim (DE)**
Erfinder : **Eicken, Karl, Dr.**
**Am Huettenwingert 12**
**W-6706 Wachenheim (DE)**
Erfinder : **Westphalen, Karl-Otto, Dr.**
**Mausbergweg 58**
**W-6720 Speyer (DE)**
Erfinder : **Wuerzer, Bruno, Dr.**
**Ruedigerstrasse 13**
**W-6701 Otterstadt (DE)**

EP 0 456 125 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft N-Aryltetrahydrophthalimide der allgemeinen Formel I

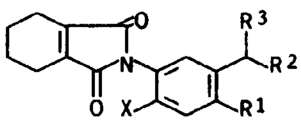

I

in der die Substituenten folgende Bedeutung haben:

X Wasserstoff oder Halogen;

$R^1$ Wasserstoff, Halogen oder $C_1$-$C_4$-Alkyl, welches ein bis fünf Halogenatome tragen kann;

$R^2$ ein 5-Ring Heteroaromat, enthaltend neben Kohlenstoffringgliedern ein bis drei Stickstoffatome, wobei dieser Heterocyclus über ein Stickstoffatom gebunden ist;

$R^3$ Halogen, $C_1$-$C_4$-Alkylthio,
ein 5-Ring Heteroaromat, enthaltend neben Kohlenstoffringgliedern ein bis drei Stickstoffatome, wobei dieser Heterocyclus über ein Stickstoffatom gebunden ist oder
die Phenylthiogruppe, die ihrer noch einen bis drei der folgenden Reste tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio, und wobei die Phenylthiogruppe zusätzlich noch so viele Halogenatome tragen kann, daß die Gesamtzahl der Reste 4 oder 5 beträgt;

sowie deren landwirtschaftlich nutzbaren Salze.

Des weiteren betrifft die Erfindung Verfahren zur Herstellung dieser Verbindungen sowie sie enthaltende herbizide Mittel.

Aus der Literatur (EP-A-207 894, EP-A-319 791 und EP-A-0 337 151) sind N-(Phenyl)-3,4,5,6-tetrahydrophthalimide als herbizid wirksame Verbindungen bekannt. Die Herstellung von N-(3-Formyl-phenyl)-3,4,5,6-tetrahydrophthalimiden ist in der DE-A-38 15 042 beschrieben.

Die Selektivität der bekannten herbizid wirksamen Tetrahydrophthalimide bezüglich der Schadpflanzen vermag jedoch nur bedingt zu befriedigen. Der Erfindung lagen deshalb neue herbizid wirksame Verbindungen als Aufgabe zugrunde, mit denen sich die Schadpflanzen besser und gezielter bekämpfen lassen, ohne daß dabei die Nutzpflanzen nennenswert geschädigt werden.

Demgemäß wurden die eingangs definierten N-Aryltetrahydrophthalimide der Formel I gefunden.

Weiterhin wurden ein Verfahren zur Herstellung dieser N-Aryltetrahydrophthalimide gefunden sowie sie enthaltende herbizide Mittel.

Die Verbindungen der Formel I, in denen $R^2$ und $R^3$ verschieden sind, enthalten ein asymmetrisches C-Atom und können somit als Enantiomere auftreten. Die Racemate lassen sich nach bekannten Methoden, beispielsweise durch Salzbildung mit einer optisch aktiven Säure, trennen. Als Wirkstoffe sind sowohl die reinen Enantiomeren als auch das bei der Synthese anfallende Isomerengemisch geeignet.

Die Verbindungen I sind auf verschiedenen Wegen herstellbar. Insbesondere werden Sie nach den im folgenden beschriebenen Wegen synthetisiert.

Man erhält die Verbindungen I, in denen $R^3$ für Halogen steht, besonders vorteilhaft dadurch, daß man ein N-(3-Formylphenyl)-tetrahydrophthalimid der allgemeinen Formel II in an sich bekannter Weise (Tetrahedron Lett. 52, 5011 (1979)) in einem inerten organischen Lösungsmittel in Gegenwart eines anorganischen Säurehalogenids [Hal] mit einem Stickstoff-enthaltenden 5-Ring Heteroaromaten der allgemeinen Formel III umsetzt.

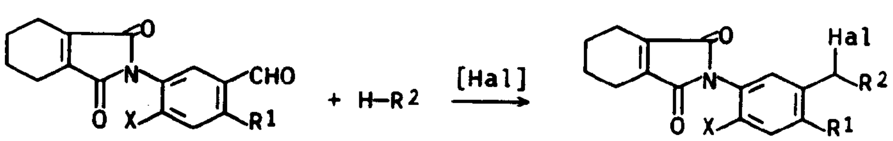


II III I ($R^3$ = Halogen)

Als anorganische Säurehalogenide [Hal] eignen sich Halogenierungsmittel wie Phosphoroxidchlorid, Thiophosgen, bevorzugt Phosgen sowie Thionylchlorid und -bromid.

Die Ausgangsverbindungen II sind bekannt oder in bekannter Weise erhältlich (DE-A 3 815 042).

Das Säurehalogenid wird bevorzugt in mindestens äquimolaren Mengen, bezogen auf das N-(3-Formylphenyl)-tetrahydrophthalimid II eingesetzt. Die Azolkomponente $R^2$-H wird z.B. in der zweifachen, bevorzugt in der 5-6fachen molaren Menge, bezogen auf das Säurehalogenid, eingesetzt.

Die Umsetzung erfolgt im allgemeinen bei Temperaturen von -30 bis +100°C, bevorzugt -10 bis +50°C, insbesondere 0 bis 20°C.

Bevorzugte Lösungsmittel sind beispielsweise Nitrile wie Acetonitril, Ether wie Tetrahydrofuran, Diethylether und Dioxan. Besonders bevorzugt werden Kohlenwasserstoffe und Chlorkohlenwasserstoffe wie Hexan, Benzol, Toluol, Methylenchlorid, Tetrachlorkohlenstoff oder Gemische der genannten Solventien.

Im allgemeinen arbeitet man bei Atmosphärendruck, sofern sich nicht wegen leichtflüchtiger Reaktionspartner ein höherer Druck, etwa bis zu 5 bar empfiehlt.

Da die Säurehalogenide und die intermediär entstehenden Zwischenverbindungen hydrolyseempfindlich sind, wird die Umsetzung im allgemeinen unter einer Schutzgasatmosphäre ($N_2$, Ar) durchgeführt.

Verbindungen der Formel I, in denen $R^3$ nicht Halogen bedeutet, erhält man beispielsweise dadurch, daß man ein N-(3-Azolylmethylphenyl)tetrahydrophthalimid der allgemeinen Formel Ia in an sich bekannter Weise in einem inerten organischen Lösungsmittel in Gegenwart einer Base mit einer Verbindung der allgemeinen Formel IV umsetzt.

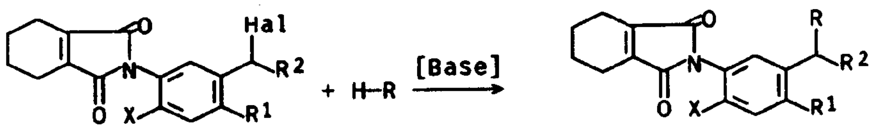


Ia ($R^3$ = Halogen) IV I ($R^3$ = R≠Halogen)

R in der Formel IV und I bedeutet eine der bei $R^3$ genannten Gruppen, jedoch nicht Halogen. Hal in der Formel Ia bedeutet Halogen wie insbesondere Chlor und Brom.

Die Verbindung IV wird vorteilhaft in stöchiometrischen Mengen, bevorzugt in ca. 20 %igem Überschuß, bezogen auf Ia, eingesetzt.

Die Umsetzung erfolgt vorteilhaft unter Zusatz einer organischen oder anorganischen Hilfsbase und/oder eines Reaktionsbeschleunigers in Gegenwart eines Lösungsmittels.

Die Menge an Base und Reaktionsbeschleuniger kann je nach der eingesetzten Verbindung variiert werden. Vorteilhaft setzt man einen geringen Überschuß an Base, bezogen auf Ia, ein.

Geeignete Basen sind beispielsweise Alkalihydroxide wie Lithium-, Natrium- oder Kaliumhydroxid, Alkalicarbonate oder -hydrogencarbonate wie Natrium- oder Kaliumcarbonat oder Natrium- oder Kaliumhydrogencarbonat, Alkaliamide wie Natrium- oder Kaliumamid sowie die organischen Basen Pyridin, 4-Dialkylaminopyridin und Dialkylaniline.

Der Reaktionsbeschleuniger wird bevorzugt in katalytischen Mengen zum Reaktionsgemisch gegeben, vorzugsweise 0,001 mol-Äq. bis 0,1 mol-Äq., insbesondere 0,01 mol-Äq. bis 0,05 mol-Äq., bezogen auf Ia.

Als Reaktionsbeschleuniger können z.B. Metallhalogenide, bevorzugt Natriumjodid oder Kaliumjodid, quartäre Ammoniumsalze wie Tetraalkylammoniumhalogenide oder -hydrogensulfate, bevorzugt Tetrabutylammoniumhalogenide wie Benzyltriethylammoniumchlorid oder -bromid und Kronenether wie 12-Krone-4, 15-Krone-5, Benzo-15-Krone-5, Dibenzo-18-Krone-6 oder Dicyclohexano-18-Krone-6, verwendet werden.

Als Lösungsmittel werden bevorzugt Ketone wie Aceton, Methylethylketon oder Cyclohexanon, Nitrile wie Acetonitril oder Propionitril, Alkohole wie Methanol, Ethanol, i-Propanol, n-Butanol, Glycole, Ester wie Essigsäuremethylester, Essigsäureethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder Gemische der genannten Lösungsmittel verwendet.

Die Umsetzung wird vorteilhaft bei Temperaturen zwischen 0 und 180°C, bevorzugt bei der Siedetemperatur des verwendeten Lösungsmittels bzw. des Gemisches durchgeführt.

Bezüglich des Druckes gelten die Angaben für die Herstellung der Verbindung Ia.

Die erfindungsgemäßen Verfahren zur Herstellung substituierter N-Aryltetrahydrophthalimide der Formel I können kontinuierlich oder diskontinuierlich durchgeführt werden.

Als landwirtschaftlich brauchbare Salze der Verbindungen I eignen sich die Salze von solchen Säuren, welche die herbizide Wirkung von I nicht beeinträchtigen, also z.B. die Hydrochloride und -bromide, Sulfate, Nitrate, Phosphate, Oxalate oder die Dodecylbenzolsulfonate sowie Metallkomplexe wie die Komplexe des Kupfers, Zinks, Zinns, Mangans, Eisens, Kobalts oder Nickels. Vorzugsweise stellt man die Komplexe aus den freien Stickstoffbasen 1 mit mineralsauren Salzen, beispielsweise den Chloriden oder Sulfaten, der entsprechenden Metalle her.

Im Hinblick auf die biologische Wirksamkeit sind N-Aryltetrahydrophthalimide I von Bedeutung, in denen die Substituenten folgende Bedeutung haben:

X Wasserstoff oder Halogen wie Fluor, Chlor, Brom und Jod, insbesondere Fluor und Chlor;

$R^1$ verzweigtes oder unverzweigtes Alkyl wie Methyl, Ethyl, Propyl, 1-Methylethyl, Butyl, 1-Methylpropyl, 2-Methylpropyl und 1,1-Dimethylethyl, insbesondere Methyl, Ethyl, Propyl und 1-Methylethyl; Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Trifluormethyl; oder eine der bei X genannten Gruppen, insbesondere Wasserstoff, Fluor, Chlor und Brom;

$R^2$ ein über sein Stickstoffatom gebundener 5-gliedriger Heteroaromat mit ein bis drei Stickstoffatomen wie 1,2-Diazol-1-yl, 1,3-Diazoyl-1-yl, 1,2,4-Triazol-1-yl, 1,3,4-Triazol-1-yl und 1-Pyrrolyl;

$R^3$ Halogen wie bei X genannt, insbesondere Chlor und Brom; Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio, Ethylthio, 1-Methylethylthio und 1,1-Dimethylethylthio;
eine der bei $R^2$ genannten Gruppen, insbesondere 1,2-Diazol-1-yl oder Phenylthio, wobei der Phenylring seinerseits noch einen bis drei der folgenden Reste tragen kann: Halogen wie bei X genannt, insbesondere Fluor, Chlor und Brom;
unverzweigtes oder verzweigtes Alkyl wie bei $R^1$ genannt, insbesondere Methyl, Ethyl und 1-Methylethyl;
partiell oder vollständig halogeniertes Alkyl wie bei $R^1$ genannt, insbesondere Difluormethyl und Trifluormethyl;
Alkoxy wie Methoxy, Ethoxy, Propyloxy, 1-Methylethoxy, Butyloxy, 1-Methylpropyloxy, 2-Methylpropyloxy und 1,1-Dimethylethoxy, insbesondere Methoxy, Ethoxy und 1-Methylethoxy;
partiell oder vollständig halogeniertes Alkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und Pentafluorethoxy; Alkylthio wie vorstehend genannt, insbesondere Methylthio und Ethylthio, und wobei die Phenylgruppe zusätzlich noch so viele Halogenatome wie vorstehend genannt, insbesondere Fluor und Chlor, tragen kann, daß die Gesamtzahl der Reste 4 oder 5 beträgt.

Beispiele für insbesondere bevorzugte N-Aryltetrahydrophthalimide der Formel I sind in der nachstehenden Tabelle wiedergegeben.

Tabelle

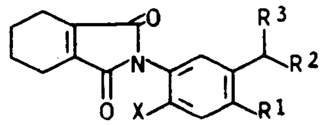

I

| X | $R^1$ | $R^2$ | $R^3$ |
|---|---|---|---|
| H | Cl | 1,2,4-Triazolyl | Cl |
| H | Cl | 1,2,4-Triazolyl | $SCH_3$ |
| H | Cl | 1,2,4-Triazolyl | Br |
| H | Cl | 1,2,4-Triazolyl | $SC_4H_9$ |
| H | Cl | 1,2,4-Triazolyl | $SC_6H_5$ |
| H | Cl | 1,2,4-Triazolyl | 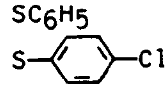 |
| H | Cl | 1,2,4-Triazolyl | 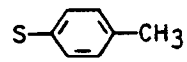 |
| H | Cl | 1,2,4-Triazolyl | 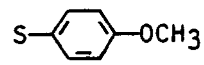 |
| H | Cl | 1,2,4-Triazolyl | 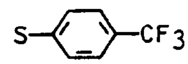 |
| H | Cl | 1,2,4-Triazolyl | Pyrazolyl |
| F | Cl | 1,2,4-Triazolyl | Cl |
| F | Cl | 1,2,4-Triazolyl | Br |
| F | Cl | 1,2,4-Triazolyl | $SCH_3$ |
| F | Cl | 1,2,4-Triazolyl | $SC_4H_9$ |
| F | Cl | 1,2,4-Triazolyl | $SC_6H_5$ |
| F | Cl | 1,2,4-Triazolyl | 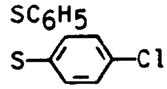 |
| F | Cl | 1,2,4-Triazolyl | 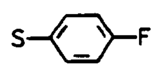 |
| Cl | Cl | 1,2,4-Triazolyl | Cl |
| Cl | Cl | 1,2,4-Triazolyl | Br |
| Cl | Cl | 1,2,4-Triazolyl | $SCH_3$ |
| F | $CF_3$ | 1,2,4-Triazolyl | Br |
| Cl | H | 1,2,4-Triazolyl | Cl |
| Cl | H | 1,2,4-Triazolyl | Br |
| Cl | F | 1,2,4-Triazolyl | Cl |
| Cl | F | 1,2,4-Triazolyl | Br |
| Cl | $CH_3$ | 1,2,4-Triazolyl | Cl |

Tabelle - Forts.

| X | $R^1$ | $R^2$ | $R^3$ |
|---|---|---|---|
| Cl | $CH_3$ | 1,2,4-Triazolyl | Br |
| H | H | Imidazolyl | Cl |
| H | H | Imidazolyl | Br |
| H | F | Imidazolyl | Cl |
| H | F | Imidazolyl | Br |
| H | Cl | Imidazolyl | Cl |
| H | Cl | Imidazolyl | Br |
| H | Cl | Imidazolyl | $SCH_3$ |
| H | $CH_3$ | Imidazolyl | Cl |
| H | $CF_3$ | Imidazolyl | Cl |
| F | H | Imidazolyl | Cl |
| F | F | Imidazolyl | Cl |
| F | Cl | Imidazolyl | Cl |
| F | $CH_3$ | Imidazolyl | Cl |
| F | $CF_3$ | Imidazolyl | Cl |
| Cl | H | Imidazolyl | Cl |
| Cl | F | Imidazolyl | Cl |
| Cl | Cl | Imidazolyl | Cl |
| Cl | $CH_3$ | Imidazolyl | Cl |
| H | H | Pyrazolyl | Cl |
| H | F | Pyrazolyl | Cl |
| H | F | Pyrazolyl | Pyrazolyl |
| H | Cl | Pyrazolyl | Pyrazolyl |
| H | $CH_3$ | Pyrazolyl | Pyrazolyl |
| H | $CF_3$ | Pyrazolyl | Pyrazolyl |
| F | H | Pyrazolyl | Pyrazolyl |
| F | F | Pyrazolyl | Pyrazolyl |
| F | Cl | Pyrazolyl | Pyrazolyl |
| F | $CH_3$ | Pyrazolyl | Pyrazolyl |
| F | $CF_3$ | Pyrazolyl | Pyrazolyl |
| Cl | H | Pyrazolyl | Pyrazolyl |
| Cl | F | Pyrazolyl | Pyrazolyl |
| Cl | Cl | Pyrazolyl | Pyrazolyl |
| Cl | $CH_3$ | Pyrazolyl | Pyrazolyl |

Die N-Aryltetrahydrophthalimide I eignen sich als Herbizide, insbesondere zur Bekämpfung von Pflanzenarten aus der Familie der Gramineen (Gräser).

Die Verbindungen I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.007 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 1.003 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.002 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.008 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.001 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.006 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.003 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.005 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an herbizidem Wirkstoff betragen je nach Bekämpfungsziel, Jahreszeit, Zielpflanzen und Wachstumsstadium 0,001 bis 3,0, vorzugsweise 0,01 bis 2,0 kg/ha aktive Substanz (a.S.).

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |

| Botanischer Name | Deutscher Name |
|---|---|
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Zea mays | Mais |

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die

Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der nachstehenden Tabelle mit physikalischen Angaben aufgeführt.

Beispiel 1

N-[3-(Chlor-1,2,4-triazol-1-ylmethyl)-4-chlorphenyl]-3,4,5,6-tetrahydrophthalimid

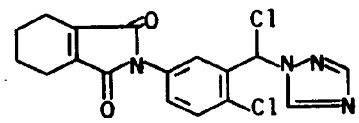

Eine Lösung aus 14,3 g Triazol in 100 ml Methylenchlorid wurde bei 0°C unter Stickstoffatmosphäre mit 6,2 g Thionylchlorid und nach 30 minütigen Rühren bei 25°C mit 10 g N-(3-Formyl-4-chlorphenyl)-3,4,5,6-tetrahydrophthalimid (0,027 mol) versetzt.

Nach 12-stündiger Reaktionszeit bei 25°C wurden 100 ml Wasser zugegeben, wonach die abgetrennte wässrige Phase zweimal mit Methylenchlorid extrahiert wurde. Die vereinigten organischen Phasen wurden sodann wie üblich auf das Azolylmethylderivat aufgearbeitet. Nach Umkristallisation aus Methyl-tert.-butylether wurden 9,7 g Produkt erhalten.
Ausbeute: 97 %; Fp.: 128-131°C
(Wirkstoffbeispiel 1.001)

Beispiel 2

N-[3-(Methylthio-1,2,4-triazol-1-ylmethyl)-4-chlorphenyl]-3,4,5,6-tetrahydrophthalimid

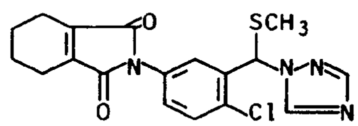

Eine Lösung aus 3 g (0,008 mol) N-[3-(Chlor-1,2,4-triazol-1-ylmethyl)-4-chlorphenyl]-3,4,5,6-tetrahydrophthalimid (Beispiel 1) in 50 ml N,N-Dimethylformamid, 0,6 g Natriummethanthiolat und einer Spatelspitze Kaliumjodid wurde 12 Stunden auf 50°C erhitzt und anschließend mit 100 ml Wasser versetzt. Nach Extraktion mit Methyl-tert.-butylether wurde die organische Phase gewaschen und wie üblich aufgearbeitet.
Ausbeute: 1,0 g (32 %); Öl
(Wirkstoffbeispiel 1.002)

Tabelle 1

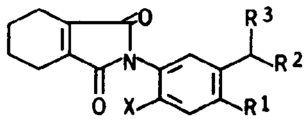

I

| Beispiel Nr. | X | $R^1$ | $R^2$ | $R^3$ | phys. Daten [Fp (°C); IR ($cm^{-1}$)] |
|---|---|---|---|---|---|
| 1.001 | H | Cl | 1,2,4-Triazolyl | Cl | 128-131 |
| 1.002 | H | Cl | 1,2,4-Triazolyl | $SCH_3$ | 1713, 1480, 1377 $cm^{-1}$ |
| 1.003 | H | Cl | 1,2,4-Triazolyl | Br | 120-124 |
| 1.004 | H | Cl | Imidazolyl | Cl | 198-204 |
| 1.005 | H | F | Pyrazolyl | Pyrazolyl | 92- 94 |
| 1.006 | H | Cl | Pyrazolyl | Pyrazolyl | 178-180 |
| 1.007 | F | F | Pyrazolyl | Pyrazolyl | 205-207 |
| 1.008 | F | Cl | Pyrazolyl | Pyrazolyl | 192-194 |

Anwendungsbeispiele

Die herbizide Wirkung der N-Aryltetrahydrophthalimide der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,125 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Abutilon theophrasti | Chinesischer Hanf |
| Chenopodium album | Weißer Gänsefuß |
| Zea mays | Mais |

Mit 0,125 kg/ha a.S. im Nachauflaufverfahren eingesetzt, lassen sich mit den Beispielen 1.002 und 1.008 breitblättrige unerwünschte Pflanzen sehr gut bekämpfen. Beispiel 1.002 zeigt gleichzeitig gute Verträglichkeit an der Beispielkultur Mais.

## Patentansprüche

1. N-Aryltetrahydrophthalimide der allgemeinen Formel I

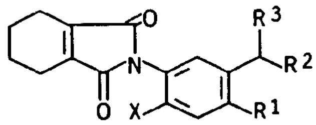

I

in der die Substituenten folgende Bedeutung haben:

X Wasserstoff oder Halogen;

$R^1$ Wasserstoff, Halogen oder $C_1$-$C_4$-Alkyl, welches ein bis fünf Halogenatome tragen kann;

$R^2$ ein 5-Ring Heteroaromat, enthaltend neben Kohlenstoffringgliedern ein bis drei Stickstoffatome, wobei dieser Heterocyclus über ein Stickstoffatom gebunden ist;

$R^3$ Halogen, $C_1$-$C_4$-Alkylthio, ein 5-Ring Heteroaromat, enthaltend neben Kohlenstoffringgliedern ein bis drei Stickstoffatome, wobei dieser Heterocyclus über ein Stickstoffatom gebunden ist oder die Phenylthiogruppe, die ihrerseits noch einen bis drei der folgenden Reste tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio, und wobei die Phenylthiogruppe zusätzlich noch so viele Halogenatome tragen kann, daß die Gesamtzahl der Reste 4 oder 5 beträgt;

sowie deren landwirtschaftlich nutzbaren Salze.

2. Verfahren zur Herstellung der Verbindungen I gemäß Anspruch 1, in denen $R^3$ für Halogen steht, dadurch gekennzeichnet, daß man ein N-(3-Formylphenyl)-tetrahydrophthalimid der allgemeinen Formel II

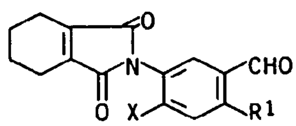

II

in an sich bekannter Weise in einem inerten organischen Lösungsmittel in Gegenwart eines anorganischen Säurehalogenids mit einem Stickstoffhaltigen 5-Ring Heteroaromaten der allgemeinen Formel III

$H$-$R^2$ III

umsetzt,
und die Verfahrensprodukte gewünschtenfalls in ihre landwirtschaftlich nutzbaren Salze überführt.

3. Verfahren zur Herstellung der Verbindungen I gemäß Anspruch 1, in denen $R^3$ nicht für Halogen steht, dadurch gekennzeichnet, daß man ein N-(3-Azolylmethylphenyl)-tetrahydrophthalimid der allgemeinen Formel Ia

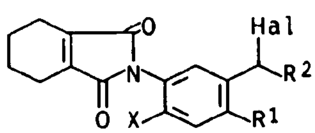

Ia

in der Hal für Halogen steht, in an sich bekannter Weise in einem inerten organischen Lösungsmittel in Gegenwart einer Base mit einer Verbindung der allgemeinen Formel IV

H-R IV

in der R eine der bei $R^3$ genannten Gruppen, jedoch nicht Halogen bedeutet, umsetzt,
und die Verfahrensprodukte gewünschtenfalls in ihre landwirtschaftlich nutzbaren Salze überführt.

4. Herbizide Mittel, enthaltend inerte Zusatzstoffe und eine herbizid wirksame Menge mindestens eines N-Aryltetrahydrophthalimids der Formel I gemäß Anspruch 1.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines N-Aryltetrahydrophthalimids der Formel I gemäß Anspruch 1 behandelt.

## Claims

1. An N-aryltetrahydrophthalimide of the formula I

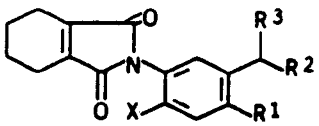

I

where
X is hydrogen or halogen,
$R^1$ is hydrogen, halogen or $C_1$-$C_4$-alkyl which may carry from one to five halogen atoms;
$R^2$ is a heteroaromatic structure having a 5-membered ring and containing, in addition to carbon ring members, from one to three nitrogen atoms, this heterocyclic structure being bonded via a nitrogen atom, and
$R^3$ is halogen, $C_1$-$C_4$-alkylthio,
a heteroaromatic structure having a 5-membered ring and containing, in addition to carbon ring members, from one to three nitrogen atoms, this heterocyclic structure being bonded via a nitrogen atom, or
phenylthio, which in turn may also carry from one to three of the following radicals: halogen, $C_1$-$C_4$-alkyl, $C_1$- $C_4$-haloalkyl, $C_1$-$C_4$ -alkoxy, $C_1$-$C_4$-haloalkoxy or $C_1$-$C_4$-alkylthio, and where the phenylthio group may also carry halogen atoms such that the total number of the radicals is 4 or 5,
and salts thereof which can be used in agriculture.

2. A process for the preparation of a compound I as claimed in claim 1, in which $R^3$ is halogen, wherein an N-(3-formylphenyl)-tetrahydrophthalimide of the formula II

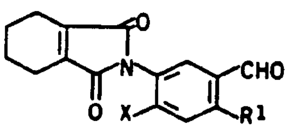

II

is reacted with a nitrogen-containing heteroaromatic structure, having a 5-membered ring, of the formula III

H-$R^2$ III

in a conventional manner in an inert organic solvent in the presence of an inorganic acid halide, and if desired the product of this process is converted into a salt thereof which can be used in agriculture.

3. A process for the preparation of a compound I as claimed in claim 1, in which $R^3$ is not halogen, wherein an N-(3-azolylmethylphenyl)-tetrahydrophthalimide of the formula Ia

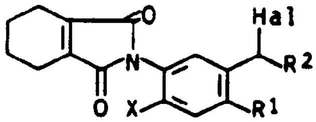

Ia

in which Hal is halogen, is reacted with a compound of the formula IV

H-R IV

where R is one of the groups stated for $R^3$ but not halogen, in a conventional manner in an inert organic solvent in the presence of a base, and if desired the product of this process is converted into a salt thereof which can be used in agriculture.

4. A herbicide containing a herbicidal amount of one or more N-aryltetrahydrophthalimides of the formula I as claimed in claim 1 and inert additives.

5. A method for controlling undesirable plant growth, wherein the undesirable plants or their habitat are or is treated with a herbicidal amount of an N-aryltetrahydrophthalimide of the formula I as claimed in claim 1.

## Revendications

1. N-aryltétrahydrophtalimides de la formule générale I

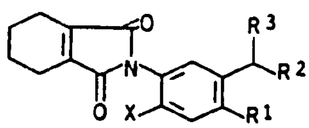

I

dans laquelle les substituants ont les significations suivantes:

- X hydrogène ou halogène
- $R^1$ hydrogène, halogène ou alkyle en C1-C4, qui peut porter un à cinq atomes d'halogène
- $R^2$ un hétéroaromate pentagonal, contenant, outre des chaînons de cycle carboné, un à trois atomes d'azote, cet hétérocyle étant lié par un atome d'azote
- $R^3$ halogène, alkylthio en C1-C4, un hétéroaromate pentagonal, contenant, outre des chaînons de cycle carboné, un à trois atomes d'azote, cet hétérocycle étant lié par un atome d'azote, ou le groupe phényle qui, de son côté, peut porter un à trois des restes suivants: halogène, alkyle en C1-C4, halogénalkyle en C1-C4, alcoxy en C1-C4, halogénalcoxy en C1-C4 ou alkylthio en C1-C4 et le groupe phénylthio pouvant porter additionnellement encore autant d'atomes d'halogène que le nombre total des restes est 4 ou 5 ainsi que leurs sels utilisables en agriculture.

2. Procédé de préparation des composés I selon la revendication 1, dans lesquels $R^3$ est mis pour halogène, caractérisé par le fait que l'on fait agir un N-(3-formylphényl)-tétrahydrophtalimide de la formule générale II

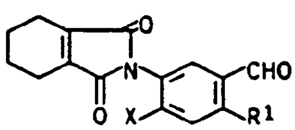

II

de manière connue en soi, dans un solvant organique inerte, en présence d'un halogènure d'acide inorganique, avec un hétéroaromate pentagonal à teneur d'azote de la formule générale III

H-$R^2$ III

et on transforme les produits du procédé, éventuellement, en leurs sels utilisables en agriculture.

3. Procédé de préparation des composés I selon la revendication 1 dans lesquels $R^3$ n'est pas mis pour halogène, caractérisé par le fait que l'on fait réagir un N-(3-azolylméthylphényl)-tétrahydrophtalimide de la formule générale Ia

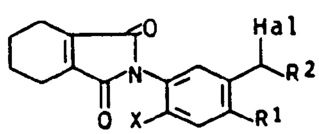

Ia

dans laquelle Hal est mis pour halogène, de manière connue en soi, dans un solvant organique inerte, en présence d'une base, avec un composé de la formule générale IV

H-R IV

dans laquelle R représente un des groupes désignés par $R^3$ mais toutefois pas halogène
et on transforme les produits du procédé, éventuellement, en leurs sels utilisables en agriculture.

**4.** Agent herbicide, contenant des additifs inertes et une quantité efficace comme herbicide d'au moins un N-aryltétrahydrophtalimide de la formule I selon la revendication 1.

**5.** Procédé pour lutter contre une croissance des plantes indésirable, caractérisé par le fait que l'on traite les plantes indésirables et/ou leur biotope avec une quantité efficace comme herbicide d'au moins un N-aryltétrahydrophtalimide de la formule I selon la revendication 1.